# EUROPEAN PATENT APPLICATION

(11) **EP 1 340 429 A1**
(43) Date of publication of application: **03.09.2003**
(21) Application number: 01982758.3
(22) Date of filing: 12.11.2001
(51) Int. Cl.: A23L 1/20, A23L 1/30, A23C 11/10

(54) **SOYBEAN MILKS CONTAINING EPA AT HIGH CONCENTRATION AND PROCESS FOR PRODUCING THE SAME**

(30) Priority: 13.11.2000 JP 2000344863
(71) Applicant: Nippon Suisan Kaisha, Ltd., Tokyo 100-0004 (JP)
(72) Inventor: NAKAJIMA, Shuji, c/o Nippon Suisan Kaisha Ltd, Tokyo 100-0004 (JP); YAMAGISHI, Yoko, c/o Nippon Suisan Kaisha Ltd, Tokyo 100-0004 (JP); HATA, Kazuhiko, c/o Nippon Suisan kaisha Ltd, Hachioji-Shi, Tokyo 192-0906 (JP)
(74) Representative: Leson, Thomas Johannes Alois, Dipl.-Ing.
(86) International application number: PCT/JP01/09878
(87) International publication number: WO 02/037985

(57) **Abstract**

The invention provides a soybean milk product, which contains fish-oil-originated EPA and/or DHA at a high content, is effective to hyperlipemia, and has a high nutritive. In the soybean milk product, triglyceride or ethyl ester containing 10 % or more of EPA is mixed in soybean milk in amount of more than 25% of the soybean protein in the soybean milk. The soybean milk product is produced by preparing soybean milk through a series of steps, and then adding and mixing a fish oil in the prepared soybean milk. The invention also provides a food product to be taken in by people as functional foods being effective to hyperlipemia. The triglyceride is a fish oil and preferably a purified fish oil. The fish oil contains EPA and DHA in adjusted amount, more specifically EPA and DHA in total amount of 25 % or more. Preferably. the fish oil contains 28 % or more of EPA. The soybean milk is soybean milk containing 3 % or more of solid part and selected from among adjusted soybean milk, a beverage containing soybean milk, acidified soybean milk, and Tofu obtained by curding soybean milk.

## Description

### FIELD OF THE INVENTION

The present invention relates to a soybean milk product containing EPA at a high content, and more particularly to a soybean milk product, which is effective to hyperlipemia and has a high nutritive value, and a method of producing the soybean milk product.

### BACKGROUND ART

Soybean milk is employed not only as materials of Tofu (bean curd), but also as beverages that are directly taken by people as functional foods in recent years. The soybean milk is usually produced as follows. First, soybeans are washed with water for removal of dust and so on. Then, the soybeans are soaked in water at the normal temperature of volume three times as much as the soybeans, and are held in the soaked condition for 5 to 20 hours depending on the season. During such a period, imbibition and germination of the soybeans occurs. Then, the imbibed soybeans are put in a grinding device, such as a mixer and a mortar, and are ground at the normal temperature while water at the normal temperature is added in volume about 5 to 6 times as much as the soybeans. Raw soybean juice (or soybean juice) is thereby produced. After heating the raw soybean juice for about 3 to 15 minutes, it is subjected to a solid-liquid separation process at high temperatures using a solid-liquid separation device, such as a centrifugal separator and a filter press. As a result, soybean milk and bean curd lees are obtained.

The soybean milk thus produced is provided as Tofu after being added with a coagulant, e.g., bittern, gluconolactone and calcium sulfate, or as beverages after being directly packed in a container and then sealed off. However, beverages containing soybean milk have unpleasant tastes, such as green grass smell and a bitter taste, due to the presence of small amounts of ill-smelling components including 2-hexenal and several kinds of saponins, and those unpleasant tastes have hitherto invited the biggest difficulty in utilization of the soybean milk. Various proposals on lactic bacteria fermentation using lactic bacteria have been made for a taste improvement of the soybean milk. But those proposals for masking the unpleasant odor of a soy bean cancel a body taste and Umami (savory taste) specific to the soy bean. Therefore, the problem of the unpleasant odor and taste specific to the soy bean is not yet overcome at the present.

It is known that soybean protein has an effect of reducing cholesterol. That fact has increased the number of people eating Tofu recently. However, containers, sauces and spices are required when eating Tofu, and places where people can eat Tofu are limited. On the other hand, when beverages containing soybean milk are packed in containers and sealed off in a portable way, there are no limitations on places for people to drink the packed beverages and people can easily take in the soybean milk as functional foods anywhere.

Meanwhile, with recent widespread diffusion of preventive medicine knowledge, it has become a prevailing practice for an increasing number of people to preventively take natural origin foods, which have physiological activities, in a healthy or half-healthy condition before they suffer from diseases, for the purpose of keeping themselves healthy. Among those foods, fish such as a horse mackerel, bonito, mackerei and sardine contains a small amount of effective natural compounds, i.e., polyunsaturated fatty acids represented by eicosapentaenoic acid (EPA) and docosahexaenoic acid (DHA), in the form of glycerides. Those natural compounds have lately received keen attention because useful physiological activities, such as a cerebral nerve activation action, a blood cholesterol and lipid lowering effect, and an antiallergic effect, have been found therein one after another in recent years. It has therefore been proposed to not only take those natural compounds as functional foods, but also to mix them in various foods.

EPA and DHA are typical ones of polyunsaturated fatty acids and are relatively abundantly contained in fat of fish (referred to as a "fish oil" hereinafter), such as fatty meat of a horse mackerel, salmon roe, yellow tail, mackerei, saury, eel, pilchard, rainbow trout, salmon, and saurel. EPA and DHA contained in the fish oil have physiological activity effects, such as a platelet aggregation inhibitory effect, a blood triglyceride lowering effect, and a blood VLDL and LDL cholesterol lowering effect, and hence have preventive and remedial effects for arteriosclerotic diseases. It is also known that depletion of DHA lowers the memory learning ability. While EPA and DHA have, on one side, have many physiological activity effects mentioned above, the fish oil containing EPA and DHA has, on the other side, a peculiar foul odor. Also, those polyunsaturated fatty acids are very easily oxidized because EPA has twenty carbons and five double bonds in a molecule and DHA has twenty-two carbons and six double bonds in a molecule. It is further known that the EPA and DHA generate an unpleasant odor and taste with deterioration of the specific taste.

Heretofore, various types of soybean milk products have been proposed in consideration of the state of the art described above. As one example of the prior art, Japanese Patent Laid-Open Publication No. 7-255406 discloses a method of producing soy bean processed food enriched with polyunsaturated fat, wherein soybean milk containing polyunsaturated fat is produced by mixing soybean milk and about 1 % to about 25 % of polyunsaturated fat with respect to the weight of soybean protein in the soybean milk. According to the detailed description in the Publication, "a maximum amount of EPA(DHA)-containing liquid fat uniformly dispersible in 18 liters of soybean milk (about 650 g of protein content) is 160 g (about 25 % with respect to the weight of protein in the soybean milk)", and "if the fat content exceeds about 25 %, the fat is not uniformly dispersed and an oil component is separated".

As another example of the prior art, Japanese Patent Laid-Open Publication No. 10-42819 discloses "a method of producing a beverage containing DHA-mixed soybean milk, the method comprising the steps of adding and mixing soybean milk and a DHA-containing fish oil at a weight ratio of 1 : 0.2 to 1, thereby preparing an emulsion of the DHA-containing fish oil, and further diluting the emulsion with the soybean milk".

### DISCLOSURE OF THE INVENTION

An object of the present invention is to provide a soybean milk product containing fish-oil-originated EPA and/or DHA at a high content, and more particularly to a soybean milk product, which is effective to hyperlipemia and has a high nutritive value, and a method of producing the soybean milk product.

According to the guideline of an intake specified by JHNA (Japan Health Food & Nutrition Food Association), the required amount of EPA is 750 mg/day in terms of EPA-TG and 1800 mg/day in terms of ethyl ester as a medicine. The above-cited Japanese Patent Laid-Open Publication No. 7-255406 does not disclose the kind of polyunsaturated fat used for producing the soybean milk, the content of EPA and DHA mixed in foods is usually about 25 % at maximum. Based on such a value, the EPA content in the soybean milk is calculated as about 0.2 %. It is hence cannot be said that the EPA content in the soybean milk satisfies the intake per day sufficiently. Considering, as one practical example, the case where a fish oil having the EPA content of 28 % is mixed in the soybean milk having 8 % of solid part, a maximum limit in addition of EPA in the prior art is about 280 mg/100 ml on condition that the content of soybean protein in the solid part is about 50 %. The present invention is intended to enable the soybean milk to contain EPA in a larger amount than that maximum limit.

Looking at the condition of patients diagnosed as hyperlipemia, the amount of only triglyceride increases in some ones, the amount of only cholesterol increases in other ones, and the amounts of both triglyceride and cholesterol increase in still other ones. In development of functional foods effective to hyperlipemia, therefore, those functional foods are desirably effective to any types of patients.

It is known that soybean protein is effective in suppressing a high cholesterol condition and EPA is effective in suppressing a high triglyceride condition. To produce functional foods from soybean protein practically, however, the effectiveness, the ease in use, etc. of the functional foods greatly depend on in what form and amount EPA is mixed because EPA is easily oxidized and has a foul odor, and in what amount the soybean protein is required.

On an assumption that when people drink soybean milk as functional foods everyday, a maximum limit in amount of the soybean milk capable of being taken in by people per day is about 200 ml, and that the intake of EPA per day is not less than 900 mg in terms of fatty acid glyceride, the present invention is intended to realize a soybean milk product containing soybean protein and EPA at required contents in the soybean milk of not more than 200 ml or not more than 125 ml. Further, the present invention is intended to provide a soybean milk product, to be taken in as functional foods having a superior nutritive value, wherein an appropriate amount of purified fish oil containing EPA is dispersed and dissolved in the soybean milk.

In addition, the present invention is intended to provide a soybean milk product that is expected to develop physiological activities of DHA contained therein together with EPA.

According to the present invention, the above object and intensions are achieved by providing a soybean milk product wherein triglyceride or ethyl ester containing 10 % or more of EPA is mixed in amount of more than 25 % of the soybean protein in the soybean milk.

To set forth in more detail, the present invention resides in a soybean milk product wherein triglyceride or ethyl ester containing 10 % or more of EPA is mixed in soybean milk in amount of more than 25 % of the soybean protein in the soybean milk, the soybean milk being preferably soybean milk containing 3 % or more of soybean solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the triglyceride is a fish oil. In this case, the present invention resides in a soybean milk product wherein a fish oil containing 10 % or more of EPA is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the fish oil contains EPA and/or DHA in adjusted amount. In this case, the present invention resides in a soybean milk product wherein a fish oil containing 10 % or more of EPA, or preferably a fish oil containing EPA and/or DHA in adjusted amount, is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the fish oil is a purified fish oil. In this case, the present invention resides in a soybean milk product wherein a purified fish oil containing 10 % or more of EPA, or preferably a purified fish oil containing EPA and/or DHA in adjusted amount, is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the fish oil contains EPA and DHA in total amount of 25 % or more. In this case, the present invention resides in a soybean milk product wherein a fish oil (preferably purified fish oil) containing 10 % or more of EPA and containing EPA and DHA in total amount of 25 % or more, or preferably a fish oil (purified fish oil) containing EPA and/or DHA in adjusted amount, is mixed in soybean milk in amount of more than 25% of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the fish oil contains 28 % or more of EPA. In this case, the present invention resides in a soybean milk product wherein a fish oil (preferably purified fish oil) containing 28 % or more of EPA, or preferably a fish oil (purified fish oil) containing EPA and/or DHA in adjusted amount, is mixed in soybean milk in amount of more than 25% of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the fish oil is a fish oil added with 0.5 % or more of tocopherol as an antioxidant. In this case, the present invention resides in a soybean milk product wherein a fish oil containing 10 % or more of EPA, preferably a fish oil or a purified fish oil containing EPA and/or DHA in adjusted amount, more preferably a fish oil or a purified fish oil containing EPA and DHA in total amount of 25 % or more, or even more preferably a fish oil or a purified fish oil containing 28 % or more of EPA, each of the fish oils or the purified fish oils being added with 0.5 % or more of tocopherol as an antioxidant, is mixed in soybean milk in amount of more than 25% of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Preferably, the soybean milk product is produced by preparing a soybean milk through a series of steps, and then adding and mixing a fish oil in the prepared soybean milk. In this case, the present invention resides in a soybean milk product wherein the soybean milk product is produced by preparing soybean milk through a series of steps, and then adding and mixing a fish oil in the prepared soybean milk, the fish oil being a fish oil containing 10 % or more of EPA, or a fish oil or a purified fish oil added, as required, with 0.5 % or more of tocopherol as an antioxidant, being preferably a fish oil or a purified fish oil containing EPA and/or DHA in adjusted amount, being more preferably a fish oil or a purified fish oil containing EPA and DHA in total amount of 25 % or more, or being even more preferably a fish oil or a purified fish oil containing 28 % or more of EPA, the fish oil being mixed in the soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the soybean milk product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Also, the present invention resides in a food product containing a soybean milk product, the food product being preferably functional food effective to hyperlipemia, wherein triglyceride or ethyl ester containing 10 % or more of EPA is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the triglyceride is a fish oil. In this case, the present invention resides in a food product containing a soybean milk product, the food product being preferably functional food effective to hyperlipemia, in which a fish oil containing 10 % or more of EPA is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the fish oil contains EPA and/or DHA in adjusted amount. In this case, the present invention resides in a food product containing a soybean milk product, the food product being preferably functional food effective to hyperlipemia, wherein a fish oil containing 10 % or more of EPA, or preferably a fish oil containing EPA and/or DHA in adjusted amount, is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the fish oil is a purified fish oil. In this case, the present invention resides in a food product containing a soybean milk product, the food product being preferably functional food effective to hyperlipemia, wherein a purified fish oil containing 10 % or more of EPA, or preferably a purified fish oil containing EPA and/or DHA in adjusted amount, is mixed in soybean milk in amount of more than 25 % of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the fish oil contains EPA and DHA in total amount of 25 % or more. In this case, the present invention resides in a food product containing a soybean milk product, the food product being preferably functional food effective to hyperlipemia, wherein a fish oil (preferably purified fish oil) containing 10 % or more of EPA and containing EPA and DHA in total amount of 25 % or more, or preferably a fish oil (purified fish oil) containing EPA and/or DHA in adjusted amount, is mixed in soybean milk in amount of more than 25% of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the fish oil contains 28 % or more of EPA. In this case, the present invention resides in a food product containing a soybean milk product, the food product being preferably functional food effective to hyperlipemia, wherein a fish oil (preferably purified fish oil) containing 28 % or more of EPA, or preferably a fish oil (purified fish oil) containing EPA and/or DHA in adjusted amount, is mixed in soybean milk in amount of more than 25% of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the fish oil is a fish oil added with 0.5 % or more of tocopherol as an antioxidant. In this case, the present invention resides in a food product containing a soybean milk product, the food product being preferably functional food effective to hyperlipemia, wherein a fish oil containing 10 % or more of EPA, preferably a fish oil or a purified fish oil containing EPA and/or DHA in adjusted amount, more preferably a fish oil or a purified fish oil containing EPA and DHA in total amount of 25 % or more, or even more preferably a fish oil or a purified fish oil containing 28 % or more of EPA, each of the fish oils or the purified fish oils being added with 0.5 % or more of tocopherol as an antioxidant, is mixed in soybean milk in amount of more than 25% of the soybean protein in the soybean milk; the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

In the above food product, preferably, the soybean milk product is produced by preparing soybean milk through a series of steps, and then adding and mixing a fish oil in the prepared soybean milk. In this case, the present invention resides in a food product containing a soybean milk product, the food product being preferably functional food effective to hyperlipemia, wherein the soybean milk is produced by preparing soybean milk through a series of steps, and then adding and mixing a fish oil in the prepared soybean milk, the fish oil being a fish oil containing 10 % or more of EPA, or a fish oil or a purified fish oil added, as required, with 0.5 % or more of tocopherol as an antioxidant, being preferably a fish oil or a purified fish oil containing EPA and/or DHA in adjusted amount, being more preferably a fish oil or a purified fish oil containing EPA and DHA in total amount of 25 % or more, or being even more preferably a fish oil or a purified fish oil containing 28 % or more of EPA, the fish oil being mixed in soybean milk in amount of more than 25% of the soybean protein in the soybean milk, the soybean milk being preferably soybean milk containing 3 % or more of solid part and, more specifically, being any one selected from among adjusted soybean milk, a beverage containing soybean milk, acidified soybean milk, and Tofu obtained by curding soybean milk, the food product of the present invention being preferably effective to hyperlipemia and, more specifically, being effective to any types of hyperlipemia in which the amount of only triglyceride increases, the amount of only cholesterol increases, and the amounts of both triglyceride and cholesterol increase.

Further, the present invention resides in a method of producing a soybean milk product, the method comprising the steps of preparing soybean milk through a series of steps, and then adding and mixing, in the prepared soybean milk, a fish oil containing 10 % or more of EPA in amount of more than 25% of the soybean protein in the soybean milk.

### BRIEF DESCRIPTION OF THE DRAWINGS

Fig. 1 is a graph showing a change of whole cholesterol over time resulting from a test in Example 6.
Fig. 2 is a graph showing a change of triglyceride over time resulting from the test in Example 6.

### BEST MODE FOR CARRYING OUT THE INVENTION

Soybean milk, preferably soybean milk containing 3 % or more of solid part, for use in the present invention may be obtained by any suitable method, and it can be, for example, soybean milk produced by a usual method from soybeans and/or defatted soybeans. Preferably, skin- and albumen-removed soybeans are used in point of providing soybean milk with a better taste. The soybean milk thus produced is provided as a beverage containing the soybean milk as it is, or Tofu after being added with a coagulant, e.g., bittern, gluconolactone and calcium sulfate, or fermented soybean milk (acidified soybean milk) after being subjected to lactic bacteria fermentation. Thus, the soybean milk is any one selected from among adjusted soybean milk, a beverage containing soybean milk, acidified soybean milk, and Tofu obtained by curding soybean milk.

Soybean milk can be produced by grinding whole soybeans and/or skin-removed soybeans, which are in a hydrated condition after or without being soaked in water, to obtain soybean juice, and then by removing insoluble fractions from the soybean juice through, e.g., filtration. More specifically, soybean milk is produced as follows. First, whole soybeans, skin-removed soybeans, and/or skin- and albumen-removed soybeans are contacted with warm or hot water at 50 to 100 °C to remove components which are soluble in the warm or hot water. Then, the imbibed soybeans are ground to obtain raw soybean juice (or soybean juice). The soybean juice is immediately introduced to a solid-liquid separation device, such as a centrifugal separator, for quick separation into solid and liquid parts. A filtrate (soybean milk) obtained after removing an insoluble fraction (solid part called bean curd lees) is preferably used as the soybean milk in the present invention. For grinding the imbibed soybeans, an ordinary grinding device, such as a mortar, a mixing machine and a mixer, can be used. The obtained soybean milk may be subjected to heat sterilization at 135 to 150 °C for 1 to 120 seconds, and then cooled.

In the case of fermenting soybean milk with lactic bacteria, the soybean milk can be produced by a usual method from soybeans and/or defatted soybeans, but may preferably contain 1.0 weight % or less of soluble glucose in a dry material of the soybean milk for the purpose of facilitating control of the lactic bacteria fermentation and providing a product having a fine fresh taste free from unfavorable tastes. Lactic bacteria assimilation saccharides (e.g., oligosaccharides) are not always required, but addition of those saccharides promotes the lactic bacteria fermentation and provides soybean milk having a better taste through the lactic bacteria fermentation. Lactic bacteria for use in the lactic bacteria fermentation are not limited to particular ones, but may be strains usually employed in yogurt, or may be any of combinations of known strains used for improving the taste of the soybean milk.

For example, known strains of lactic bacteria belonging to such genuses as Lactobacillus bulgaricus, Lactobacillus acidophilus, Lactobacillus casei, Streptococcus thermophilus, Streptococcus thermophilus, Streptococcus lactis, and Bifidobacterium, are usable. Those lactic bacteria can be used either alone or a combination of two or more selected from among them.

A fermentation method can be implemented by adding a bulk starter prepared in advance, or by adding frozen concentrated bacteria or freeze-dry concentrated bacteria directly in the soybean milk. Though depending on the temperature and time of a fermentation process, the amount of added bulk start is, e.g., 0.5 to 15 %, and in the case of directly adding lactic bacteria, the amount of the added lactic bacteria is selected so that the onset bacteria density is, e.g., not less than 10⁵/ml.

The lactic bacteria fermentation can be performed at the fermentation temperature of 20 to 50 °C for 3 to 48 hours and preferably 25 to 45 °C for 4 to 24 hours.

A fermentation device may be a similar one as that usually employed for producing fermented milk using milk as materials.

A soybean milk product containing EPA, which is intended by the present invention, is obtained by mixing, in soybean milk which is the filtrate (soybean milk) containing 3 % or more of solid part arid, or is acidified soybean milk produced from the filtrate through the lactic bacteria fermentation, or is Tofu obtained by curding the filtrate with addition of a coagulant, an EPA-containing purified fish oil in amount of more than 25% of the soybean protein in the soybean milk.

For increasing the EPA content in a beverage containing the soybean milk, a purified fish oil, which is to be mixed in the filtrate (soybean milk), or acidified soybean milk produced from the filtrate through the lactic bacteria fermentation, or Tofu obtained by curding the filtrate with addition of a coagulant, is preferably concentrated to such an extent that the content of EPA and/or DHA exceeds 25 %. The soybean milk produced through the lactic bacteria fermentation, to which the fat has been added, can be provided as fermented soybean milk of soft yogurt type by packing the produced soybean milk, as it is, in a container after the steps of mixing and cooling, or as fermented soybean milk of drink yogurt type by cooling the produced soybean milk after the step of homogenization. Also, various fruit types of soybean milk products can be produced by adding, as required, various flavors, pigments, stabilizers, and fruit preparations.

Also, the soybean milk produced through the lactic bacteria fermentation, which has been packed in a small container and then fermented, can be provided as lactic-bacteria fermented soybean milk of hard yogurt type by cooling it such a packed condition. In this case, various flavors, pigments, stabilizers, etc. can be freely added to the soybean milk prior to the start of the fermentation.

The triglyceride for use in the present invention can be given as, e.g., a fish oil and microbe-origin fat, and the ethyl ester for use in the present invention can be given as, e.g., one obtained through ethyl esterification of microbe-origin fat. Origins of the triglyceride and ethyl ester are not matters of problem so long as they can be used as foods.

The fish oil for use in the present invention is a fish oil containing 10 % or more of EPA, preferably a fish oil containing EPA and DHA in total amount of 25 % or more, and more preferably a fish oil containing 28 % or more of EPA. Also, the fish oil is given as a fish oil or a purified fish containing EPA and/or DHA in adjusted amount. The fish oil may be added with 0.5 % or more of tocopherol as an antioxidant.

The purified fish oil is produced through a first purification process (degumming → refining with alkali → bleaching → filtration) in which a fish oil from raw material fish, such as sardine, is processed to obtain a raw fish oil, and a second purification process (adsorption → distillation → deodorization → addition of antioxidant) in which the raw fish oil is processed into the purified fish oil. By carrying out a wintering process in addition to the usual purification process, it is possible to increase the content of polyunsaturated fatty acids such as EPA and DHA.

The purified fish oil is mixed in the soybean milk in amount of more than 25% of the soybean protein in the soybean milk so that the guideline of intake of EPA per day, i.e., 750 mg/day, is achieved. On an assumption that when people drink soybean milk as functional foods everyday, a maximum limit in amount of the soybean milk capable of being taken in by people per day is about 200 ml, and that the intake of EPA per day is not less than 900 mg in terms of fatty acid glyceride, the present invention provides a soybean milk product containing soybean protein and EPA at required contents in the soybean milk of not more than 200 ml or not more than 125 ml. Further, the present invention provide a soybean milk product, to be taken in as functional foods having a superior nutritive value, wherein an appropriate amount of purified fish oil containing EPA is dispersed and dissolved in the soybean milk. Although details of the metabolism occurred in the living body are not yet fully clarified, the physiological activities of DHA, which is present in the fish oil along with EPA, can also be expected. From this point of view, a fish oil containing EPA and DHA in total amount of 25 % or more is preferably used.

### Production Method

The soybean milk product is produced by preparing soybean milk through a series of steps, degassing the prepared soybean milk, and then mixing a fish oil in it. In a subsequent emulsifying process, to prevent the soybean milk from taking in oxygen, the emulsification is carried out under an atmosphere replaced by nitrogen, or using a mixing device of the type not allowing the soybean milk to take in air. The emulsification is preferably performed in two stages. Following the first stage of rough emulsification, the second stage of fine emulsification is performed immediately without a time lag. After the emulsification process, the soybean milk containing the fish oil is subjected to heat sterilization and packed in a container with a packing machine.

The soybean milk product of the present invention implies various types of products made of soybean milk as a main material. The soybean milk includes, for example, raw soybean milk, adjusted soybean milk obtained by processing raw soybean milk, beverages containing soybean milk, and fermented soybean milk obtained through lactic bacteria fermentation.

A food product containing the soybean milk product of the present invention implies foods that are made of soybean milk so far, foods that can be partly replaced by soybean milk without problems, and foods to which soybean milk can be added without problems. By using the soybean milk product of the present invention in the process of producing those foods, the fish oil containing EPA can be easily mixed in the foods. The foods that can be partly replaced by soybean milk include, for example, milk, yogurt, lactic bacteria drinks, and margarine. The foods to which soybean milk can be added include, for example, breads, cakes, pastes, and sauces.

Although the fish oil is mixed at a high content in the soybean milk prepared through a series of steps, oxidation resistance can be improved by carrying out the emulsification process which prevents the soybean milk from taking in oxygen. Also, by performing the two stages of emulsification, i.e., rough emulsification and fine emulsification, in succession without a time interval between them, emulsification stability can also be improved. Even in the case of using fermented soybean milk, that emulsification process enables the oil fish to be mixed in the soybean milk after being subjected to the fermentation without problems.

According to the present invention, functional foods containing EPA in an effective amount can be produced by mixing, in soybean milk containing 3 % or more of solid part (including adjusted soybean milk, beverages containing soybean milk, acidified soybean, and Tofu obtained by curding soybean milk), an EPA-containing purified fish oil in amount of more than 25% of the soybean protein in the soybean milk. In practice, when adding a fish oil having an EPA content of 28 % to general soybean milk containing 8 % or more of solid part, a maximum limit of the added EPA is about 280 mg/100 ml in the prior art. In contrast, the present invention makes it possible to add EPA to the soybean milk in amount higher than such a maximum limit, and to achieve the EPA intake of 750 mg/day that is the guideline of an EPA intake per day specified by JHNA (Japan Health Food & Nutrition Food Association).

Therefore, the present invention can provide, for example, a beverage effective to hyperlipemia, which contains EPA at a content of 900 mg/125 ml, and a sport drink containing EPA at a content of 900 mg/200 ml.

As mentioned above, the EPA intake per day recommended by JHNA is 750 mg/day. Additionally, the physiological activities of DHA, which is present in the fish oil along with EPA, can also be expected.

### Examples

The present invention will be described below in more detail in connection with Examples. Note that the present invention is not limited to the following Examples.

Fat used in Examples is a purified fish oil made by Nippon Suisan Kaisha Ltd. and has physical properties shown in Table 1 given below. This purified fish oil is produced through a first purification process (degumming → refining with alkali → bleaching → filtration) in which a fish oil from raw material fish, such as sardine, is processed to obtain a raw fish oil), a wintering process of the raw fish oil, and a second purification process (adsorption → distillation → deodorization → addition of antioxidant) in which the raw fish oil is processed into the purified fish oil.

**Table 1**

| Item | Standards | Analyzing method |
|---|---|---|
| Properties | Pale yellow liquid with no significant unpleasant taste or odor | Visual observation and sensuality evaluation |
| Fatty acid composition | | Gas chromatograph |
| EPA | 28.0 % or more | |
| DHA | 12.0 % or more | |
| Acid value | 1.00 or less | Fat analysis method |
| Peroxide value | 5.0 meq/kg or less | Fat analysis method |

### Example 1

10 Kg of a soy bean was soaked in water for 12 hours and ground after adding water in amount seven times as much as the soy bean. After separating bean curd lees, the ground soy bean was heated at 100 °C for deodorization and then subjected to centrifugal separation. By cooling a resulting liquid part, 50 liter of fresh squeezed raw soybean milk (referred to as "soybean milk" hereinafter) was obtained.

The soybean milk thus obtained was diluted with water so that the soybean milk contained 10 % of soybean solid part. A fish oil containing EPA (EPA content of 28 %) was mixed in 5 liter of the soybean milk so that the soybean milk contained 1.8 % of the fish oil. The mixture was mixed using a homo-mixer and a homegenizer having specifications listed respectively in (1) and (2) below, whereby soybean milk containing EPA (EPA content of 0.5 %) was obtained.

### (1) Homo-mixer (pre-emulisification)

Maker: Tokushu Kika K.K.
Type: 15H
No. : 2775
Head: diameter of 15 cm
Number of revolutions: 5,000 r.p.m. 5 minutes

### (2) Homegenizer

Maker: Sanwa Machine K.K.
No. : 2282
Capacity: 500 L/h (medium type)
Pressure: 150 kg two stages (primary 100 kg + secondary 50 kg)

### Example 2

The soybean milk prepared through the same steps as those in Example 1 was diluted with water so that the soybean milk contained 10 % of soybean solid part. A fish oil containing 28 % of EPA and 12 % of DHA, added with 0.5 % of tocopherol, was mixed in 5 liter of the soybean milk. The mixture was mixed using the homo-mixer and the homegenizer, whereby soybean milk containing EPA (EPA content of 0.5 %) was obtained.

A soybean milk product thus produced was packed in a retort pack in a sterility condition, and then left stand at 40 °C for about 3 weeks.
As a result of a sensuality test made on the product after that, any fish-origin odor did not occur and any problems were not found.

### Example 3

A sweetener, a flavor, etc. are first added to water heated to 50 - 55 °C, and the soybean milk was then added to the water. Thereafter, the purified fish oil was added to neutral soybean milk and acidified soybean milk at composition ratio listed in Table 2 given below. Respective resulting products were sealed off and preserved at the room temperature for three months.

**Table 2**

| Materials | Basic combination ratio % | (1) (Control Product) Kg | (2) (Oil fish 1.71 %) Kg | (3) (Oil fish 2.57 %) Kg |
|---|---|---|---|---|
| Soybean milk or acidified soy bean milk | 42.5 | 212.5 | 208.9 | 207.0 |
| Purified fish oil containing EPA·DHA | 0.0 | 0.0 | 8.6 | 12.9 |
| Other sweetener, flavor, etc | 4.2 | 21.2 | 20.8 | 20.6 |
| Water | 53.3 | 266.3 | 261.8 | 259.5 |
| Total | 100.0 | 500 | 500 | 500 |
| In (2) and (3), the amount of added fish oil was represented for one batch of 500 kg (common preparation liquid + fish oil). | | | | |

As a result of examining oxidation stability of the fish oil, satisfactory results for both the products. From the viewpoint of sensuality, it was confirmed that the acidified soybean milk product was superior to the neutral soybean milk product.

### Example 4

Blood cholesterol and triglyceride lowering effects of the soybean milk product of the present invention were studied by animal tests.

SD male rats of 4-week age were divided into four groups (control, fish oil, soybean milk and examination groups) each including six rats. The rats in the control group were fed in a free state with feed and water, the feed being prepared by adding 3.6 % of an olive oil to basic feed containing 0.5 % of cholesterol and 0.25 % of cholic acid of sodium. The rats in the fish oil group were fed in a free state with feed and water, the feed being prepared by adding 3.6 % of a fish oil, which contained 28 % of EPA and 12 % of DHA, to the basic feed. The rats in the soybean milk group were fed in a free state with the same feed as that given to the control group and soybean milk. The rats in the examination group were fed in a free state with the same feed as that given to the control group and the soybean milk product of the present invention, which was prepared by adding 1.8 % of a fish oil containing 28 % of EPA and 12 % of DHA to soybean milk. After two weeks, blood was drawn from the anesthetized heart of the rat in each group and measured for whole cholesterol, HDL cholesterol, and trigrylicride in plasma. Measured results are listed in Table 3 given below.

**Table 3**

| | Feed intake (mean g/day) | Soybean intake (mean ml/day) | Plasma whole cholesterol (mg/100ml) | Plasma HDL cholesterol (mg/100ml) | Plasma triglyceride (mg/100ml) |
|---|---|---|---|---|---|
| Control group | 13.8 | | 320.1±42.0 | 23.8±1.5 | 72.2±7.8 |
| Fish oil group | 13.3 | | 228.8±26.6 | 28.4±2.3 | 45.1±3.9 |
| Soybean milk group | 13.7 | 26.8 | 306.7±32.9 | 23.5±3.0 | 67.9±5.7 |
| Examination group | 13.6 | 26.5 | 211.3±25.0 | 30.3±2.9 | 42.2±4.3 |
| Each value is represented by mean ± standard deviation | | | | | |

As shown in Table 3, it was confirmed that the values of whole cholesterol and trigrylicride in plasma were apparently reduced in the examination group as compared with the control group, and that both the values were slightly lower in the examination than in the fish oil group.

### Example 5

A pre-test was conducted on the blood triglyceride lowering effect of the soybean milk product of the present invention based on a human test.

### <Production Process>

The production process in this Example is featured in performing a pre-emulsification step prior to the emulsification step for the purpose of improving a level of emulsification and providing the product superior in emulsification stability. The production process comprises the steps of preparation of soybean milk → degassing → pre-emulsification → emulsification → sterilization → packing. The pre-emulsification was first performed to such an extent that the grain size of 60 to 70 µm was obtained, and the main emulsification was then performed to such an extent that the grain size of 1 to 5 µm was obtained. (The term "grain size" mans the mean grain size measured using SALD-200VER made by Shimazu Seisakusho K.K.)

Note that by using an EPA-containing fish oil purified with the high-level purifying technique, a product being more superior in both preservability and taste can be obtained.

### 1) Details of Test

(1) Subjects: persons nearly suffering hyperlipemia (a man "S" at twenties and a man "F" at fourties)
(2) Period: 8 weeks (2 months)
(3) Inake: 600 mg/200 ml of EPA (in soybean milk) per day (no limitations on intake time)
(4) Remarks: no limitations on living style such as dietary restriction

### 2) Results

### Table 4: triglyceride value (mg/dl) for "S" and "F"

**Table 4**

| Triglyceride Value (mg/dl) | | | | | |
|---|---|---|---|---|---|
| | 0 week | 2 weeks | 4 weeks | 6 weeks | 8 weeks |
| "S" | 249 | 203 | 133 | 164 | 115 |
| "F" | 180 | 108 | 148 | 181 | 140 |

### <Review>

As a result of the pre-test, the triglyceride value was improved for both "S" and "F", who were nearly suffering hyperlipemia, just by taking in 600 mg/200 ml of EPA (in soybean milk) per day for 8 weeks (2 months) with neither limitations on intake time nor limitations on living style such as dietary restriction.

### Example 6

### Clinical Test on Healthy Volunteers

### <Sample Used>

A sample used was fermented soybean milk (one 125-ml bottle mixed with 900 mg of EPA and 350 mg of DHA) in which a purified fish oil containing eicosapentaenoic acid and docosahexaenoic acid (referred to as "EPA·DHA" hereinafter). The nutrient composition and material combination ratio of the used sample are listed in Tables 5 and 6 given below. The fermented soybean milk was a commercialized one made by Fuji Oil K.K., and the purified fish oil containing EPA·DHA was a commercialized one made by Nippon Suisan KAISHA LTD..

### <Subjects>

Eleven volunteers (mean age of 41.9 ± 8.5, male) were selected who had serum triglycride values of not less than 150 mg/dl and serum whole cholesterol values of not less than 200 mg/dl, and were living in healthy conditions.

### <Test Method>

Each of the volunteers continued drinking one bottle of a beverage mixed with the purified fish oil containing EPA·DHA (one 125-ml bottle mixed with 900 mg of EPA and 350 mg of DHA) per day for three months. After about 12 weeks from starting to take in the beverage, a change of serum fat was measured.

### <Results>

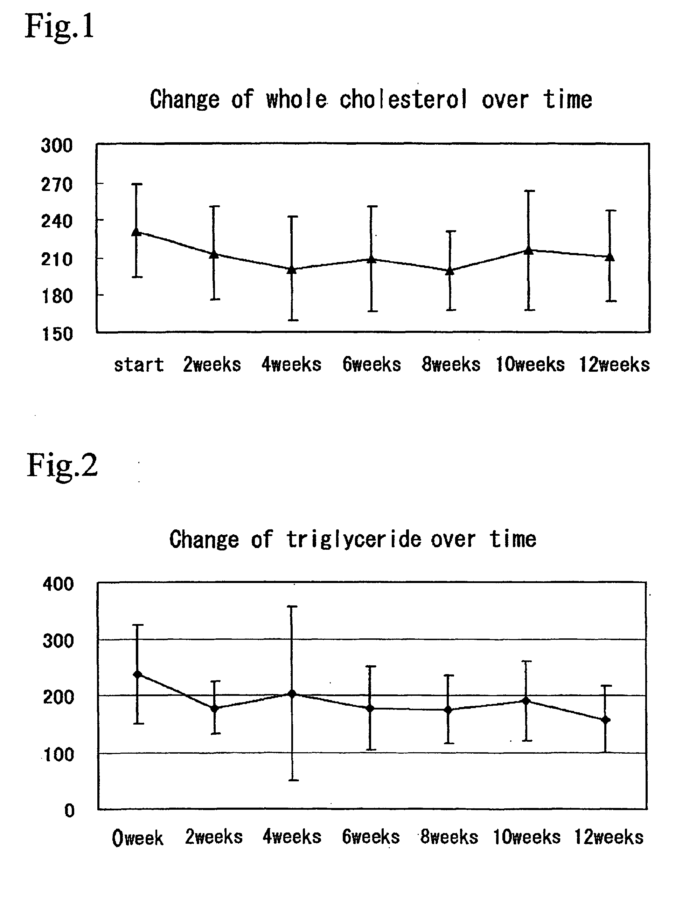

The serum whole cholesterol value was reduced from 231 ± 31 mg/dl (mean ± standard deviation, this similarly applied to values given below) at the time of starting to take in the beverage down to 211 ± 36.4 mg/dl, i.e., 8.7 %, after 12 weeks (Fig. 1). Also, the serum triglycride value was reduced from 236.8 ± 88.2 mg/dl at the time of starting to take in the beverage down to 156.8 ± 56.4 mg/dl, i.e., 33.8 %, after 12 weeks (Fig. 2). During the test period, neither problematic side effects nor drop-out examples occurred. It was hence confirmed from those results that the fermented soybean milk containing EPA are foods which people are able to drink continuously and which are useful for health care of persons having relatively high serum fat values.

**Table 5**

| Nutrient Composition of Tested Food Product | | |
|---|---|---|
| Analysis item | Unit | |
| Water | g | 108.3 |
| Protein | g | 2.0 |
| Fat | g | 5.1 |
| Ash | g | 0.1 |
| Carbohydrate | g | 0.1 |
| Energy | kcal | 79.3 |
| Sodium | mg | 21.0 |
| Eicosapentaenoic acid (EPA) | g | 0.84 |
| Docosahexaenoic acid (DHA) | g | 0.35 |

**Table 6**

| Combination Ratio of Materials | |
|---|---|
| Materials | Composition ratio |
| Fermented soybean milk | 42.4 % |
| Purified fish oil containing EPA·DHA | 2.6 % |
| Other sweetener, flavor, etc. | 4.4 % |
| Water | 50.5 % |
| | 100.0 % |

### INDUSTRIAL APPLICABILITY

According to the present invention, functional foods can be provided which enable people to take in soybean protein and EPA·DHA in respective required amounts when the people drink or eat the soybean milk product of the present invention everyday.

Also, according to the present invention, a soybean milk product containing EPA·DHA can be provided which does not cause a fish odor originating from a fish oil uses as materials, which is superior in taste, smell and texture, which shows good preservability of EPA·DHA, which and enables people to take in EPA·DHA together with soybean milk as a good protein source.

Further, according to the present invention, a stable soybean milk product containing EPA·DHA at high contents can be provided with the production process in which, to prevent the soybean milk from taking in oxygen during the production process, the emulsification is carried out under an atmosphere replaced by nitrogen, or using a mixing device of the type not allowing the soybean milk to take in air, and in which the second stage of fine emulsification is performed immediately after the first stage of rough emulsification.

## Claims

1. A soybean milk product containing triglyceride or ethyl ester, wherein the triglyceride or ethyl ester contains 10 % or more of EPA, and the amount of triglyceride or ethyl ester is more than 25% of the amount of the soybean protein in the soybean milk.

2. A soybean milk product according to Claim 1, wherein the triglyceride is a fish oil.

3. A soybean milk product according to Claim 2, wherein the contents of EPA and/or DHA in the fish oil is adjusted.

4. A soybean milk product according to Claim 2 or 3, wherein the fish oil is a purified fish oil.

5. A soybean milk product according to any one of Claims 2, 3 and 4, wherein the fish oil contains 25 % or more of total amount of EPA and DHA.

6. A soybean milk product according to any one of Claims 2 to 5, wherein the fish oil contains 28 % or more of EPA.

7. A soybean milk product according to any one of Claims 1 to 6, wherein the soybean milk is soybean milk containing 3 % or more of solid part.

8. A soybean milk product according to any one of Claims 1 to 7, wherein the soybean milk is any one selected from among adjusted soybean milk, a beverage containing soybean milk, acidified soybean milk, and Tofu obtained by curding soybean milk.

9. A soybean milk product according to any one of Claims 2 to 8, wherein the fish oil is a fish oil added with 0.5 % or more of tocopherol as an antioxidant.

10. A soybean milk product according to any one of Claims 2 to 9, wherein the soybean milk product is produced by preparing soybean milk through a series of steps, and then adding and mixing a fish oil in the prepared soybean milk.

11. A soybean milk product according to any one of Claims 1 to 10, wherein the soybean milk product is effective to hyperlipemia.

12. A soybean milk product according to Claim 11, wherein the soybean milk product is effective to any types of hyperlipemia in which the amount of only triglyceride increases, the amount of only cholesterol increases, and the amounts of both triglyceride and cholesterol increase.

13. A food product containing a soybean milk product according to any one of Claims 1 to 12.

14. A food product according to Claim 13, wherein the food product is functional food effective to hyperlipemia.

15. A method of producing a soybean milk product, comprising the steps of preparing soybean milk through a series of steps, and the step of mixing a fish oil into the prepared soybean milk, wherein the fish oil contains 10 % or more of EPA and the amount of the fish oil is more than 25 % of the soybean protein in the soybean milk.
